# EUROPEAN PATENT SPECIFICATION

(11) **EP 0 614 697 B1**
(45) Date of publication and mention of the grant of the patent: **03.02.1999**
(21) Application number: 93919620.0
(22) Date of filing: 07.09.1993
(51) Int. Cl.: B01J 20/26, B01J 20/28

(54) **COMPOSITION FOR SHEETS OF HIGH WATER ABSORPTION**
ZUSAMMENSETZUNGEN FÜR BLÄTTER MIT HOHER WASSERABSORPTION
COMPOSITION RELATIVE A DES FEUILLES A FORTE ABSORPTION D'EAU

(30) Priority: 25.09.1992 JP 256675/92; 31.05.1993 JP 129597/93
(43) Date of publication of application: 14.09.1994
(73) Proprietor: KYOWA HAKKO KOGYO CO., Ltd., Chiyoda-ku, Tokyo 100 (JP)
(72) Inventor: INABA, Yukio 2-5-45, Umaokoshi, Mie 510 (JP); YOKOMORI, Yorozu, Mie 510 (JP); KONDO, Ryuzaburo, Tokyo 156 (JP)
(74) Representative: Rauh, Peter A., Dr.
(86) International application number: JP9301263
(87) International publication number: WO9407599

(56) References cited:
- EP-A- 0 138 427
- EP-A- 0 278 601
- EP-A- 0 471 595
- WO-A-86/04910
- WO-A-90/09236
- JP-A- 1 118 546
- JP-A-63 031 539
- PATENT ABSTRACTS OF JAPAN vol. 12, no. 242 (C-510) & JP-A-63 031 539 (ASAHI)

## Description

The present invention relates to a composition for superabsorbent gelling sheets to be used for the excrement disposal in an artificial anus, a night chair and the like.

It is known that disposing of the excrement collected in an artificial anus, an artificial bladder, a urine glass, a night chair, etc. is facilitated by putting in advance superabsorbent polymers in these containers for the purpose of solidifying the excrement. (Japanese Published Unexamined Utility Model Application No. 72998/1984). A disadvantage of disposing of the excrement in this manner is that the superabsorbent polymers in the form of particles or powders become gel in the container and the gel does not fully absorb water when contacted with muddy feces containing a large amount of solids. The disposal capacity is thus unsatisfactory. In order to make up for this disadvantage, attempts have been made to develop a superabsorbent gelling sheet by the dry process. Solid superabsorbent polymers are molded into sheets by injection to prepare a superabsorbent polymer composition (Japanese Published Unexamined Patent Application Nos. 67127/1985 and 2918/1987). Solid superabsorbent polymers are sandwiched between two sheets of paper comprising water-soluble resin and pulp fibers to prepare superabsorbent sheet materials (Japanese Published Unexamined Patent Application No. 2918/1987).

A superabsorbent sheet prepared by the injection molding method is easy to cut into desirably shaped pieces and absorbs a comparatively large amount of water per unit area, but it is not so good in water absorption capacity in the initial stage. On the other hand, a superabsorbent sheet prepared by the sandwich method absorbs water rapidly in the initial stage but its water absorption capacity per unit area is not so good. Moreover, it is not so good in retaining the absorbed water in the superabsorbent polymers.

The present invention relates to a composition for superabsorbent gelling sheets which composition comprises superabsorbent polymers, fiber and polyalkylene oxide. The polyalkylene oxide bridges the space between the superabsorbent polymers and fibers so that the composition takes the form of porous sponge. The present invention also relates to a composition for superabsorbent gelling sheet which composition is obtained by supplementing the above-mentioned composition with a filter aid. Further, the present invention relates to a material for the excrement disposal, composed of the above-mentioned compositions.

An object of the present invention is to provide a composition for superabsorbent gelling sheets comprising a superabsorbent polymer, fiber and polyalkylene oxide so that the composition takes the form of a porous sponge. The composition contains the superabsorbent polymer in a high ratio so that the composition can absorb a large amount of water per unit area. Therefore, the composition is suitable for a material for the excrement disposal. Another object of the present invention is to provide a composition for superabsorbent gelling sheets which composition is prepared by supplementing said composition with a filter aid, has a remarkable water absorption capacity per unit area and has a high water absorption rate in the initial stage. The superabsorbent gelling sheets composed of said compositions can be cut into desirably shaped pieces and are suitable for a material for the excrement disposal.

The superabsorbent polymer to be used in the present invention includes, for example, crosslinked sodium polyacrylate, acrylic acid-vinyl alcohol copolymer, polyvinyl alcohol copolymer, saponified vinyl acetate-methyl acrylate copolymer, saponified vinyl acetate-monomethyl maleate copolymer, saponified isobutylene-maleic acid anhydride copolymer, crosslinked starch-acrylic acid salt graft copolymer, hydrolyzed starch-acrylonitrile graft copolymer and crosslinked modified polyethylene oxide. As the commercially available superabsorbent polymer, mention may be made of "Sumikagel" (product of Sumitomo Chemical Co., Ltd.), "Aquareserve" (product of Nippon Gosei Co., Ltd.), "Sanwet" (product of Sanyo Chemical Industries Ltd.), "Panakayaku" (product of Nippon Kayaku Co., Ltd.), "Aquakeep" (product of Sumitomo Seika Chemicals Co., Ltd.), and "WAS" (product of Nichiden Kagaku Co., Ltd.). These polymers may be used either alone or in combination.

The superabsorbent polymer should be used in an amount of 5 - 75 wt%, preferably 10 - 50 wt%, based on the total solids (hereinafter "%" means wt%). The amount of absorbed water and the water absorption behavior vary depending on the kind and the amount of the superabsorbent polymer in the composition. Therefore, the kind and amount of the superabsorbent polymer should properly be determined according to the object and conditions of use. As the polyalkylene oxide to be used in the present invention, any polyalkylene oxide can be used so long as it is thermoplastic and soluble in an organic solvent such as chloroform, methylene chloride, trichloroethylene, methyl alcohol and methyl ethyl ketone. The polyalkylene oxide includes, for example, polyethylene oxide, polyoxyethylene glycol and polyoxyethylene glycol-derived compounds. Specifically, "Paogen" is commercially available from Dai-ichi Kogyo Seiyaku Co., Ltd.

The polyalkylene oxide functions as a cross linking agent for the porous spongy product. The polyalkylene oxide is used in an amount less than 35%, preferably 25 - 10%, based on the total solids so that it has no adverse effect on water absorption but permits the sheet to absorb water rapidly in the initial stage.

As the fiber to be used in the present invention, either natural fiber or synthetic fiber can be used. Cellulosic fiber is preferred because of its good water absorption. The fiber is crushed or cut into pieces less than 5.0 mm to ensure its uniform dispersion into the spongy sheet during the making process. The fiber is preferably in a form of linter or large flakes of pulp which is/are obtained by crushing the fibers to a length of 2 - 3 mm.

The aim to incorporate the fiber into the sheet is to reinforce the function of the superabsorbent polymer and to form the porous spongy structure. The amount of the fiber is less than 20%, preferably 15 - 3%, based on the total solids in consideration of easy production of the composition. An appropriate amount varies depending on the object and conditions of use.

As the filter aid to be used in the present invention, any filter aid can be used so long as it is inert and incompressible, and is capable of forming a porous sheet. The filter aid includes, for example, diatomaceous earth, activated carbon, talc and pearlite. The filter aid is used either alone or in combination.

The filter aid is intended to improve the water absorption ability of the composition in the initial stage and to form the porous spongy structure. The filter aid facilitates the production of the superabsorbent gelling sheet because the filter aid makes the cast sheet porous and accelerates the solvent evaporation. The filter aid is added in an amount more than 0.1% based on the total solids. The amount per square meter of the cast sheet should be 0.3 - 1.5 kg, preferably 0.5 - 0.8 kg, in case of assuming a doctor blade clearance of 1 - 1.5 mm as a standard. An appropriate amount of the filter aid varies depending on the kind of solvent, the viscosity of slurry, the amount of polyalkylene oxide and the desired strength of the sheet.

The following procedure is employed to produce the composition for superabsorbent gelling sheets comprising superabsorbent polymer, fiber and polyalkylene oxide.

First, the polyalkylene oxide is dissolved in an organic solvent. To the solution is added the superabsorbent polymer and the fiber, and the mixture is stirred to give a slurry. The slurry is cast onto a flat board and the cast sheet is allowed to stand for the solvent evaporation. The solvent evaporation may be accelerated by vibrating the flat board with a vibrator attached thereto. The resulting cast sheet has two layers: the lower layer which is in contact with the flat board, and which is rich in the superabsorbent polymer and the upper layer which is rich in the napped fibers. This structure provides excellent properties required for the excrement disposal.

The above procedure may be modified such that the solution of polyalkylene oxide is incorporated with a mixture of the superabsorbent polymer and fiber, and if necessary filter aid to obtain a slurry. The resulting slurry is cast onto a flat board, followed by the solvent evaporation. The casting may be replaced by the coating with a doctor blade, in which the slurry is continuously applied to a release film, followed by the drying and peeling-off.

The composition of the present invention contains optional ingredients, such as deodorant, coloring agent and extender (e.g., calcium carbonate), in addition to the above-mentioned superabsorbent polymer resin, fibers and polyalkylene oxide. The deodorant includes, for example, plant-derived refined oil such as lime oil, bitter almond oil, lavender oil, peppermint oil, sweet orange oil, petigrain oil, eucalyptus oil and coumarin; pyrolignous acid obtained by dry distillation of wood; purified extract (Sarsaponin) from Yacca Schidigera (a species of cactus), extracts from leaves and stems, of Theaceae such as tea plant, camellia, and extracts from leaves of Quercus Salicina Blume (Fagaceae), and plant extracts containing abietic acid or phenols and aldehydes such as flavonoids, tannic acid, gallic acid, brazilin and shikonin. The deodorant is used either alone or in combination. The amount of the deodorant is 50 - 60 ml per square meter of the material for the excrement disposal. The deodorant may be sprayed onto the sheet or incorporated into the slurry prior to its casting.

The following test examples demonstrate the effect of the composition of the present invention.

### Test Example 1

Each of the compositions (Nos. 1, 2, 5 and 6) obtained in Examples was cut into five equal pieces by scissors. Each piece (containing 2g of the superabsorbent resin) was placed in a 300-ml beaker. Urine of a healthy adult was put into the beaker to evaluate the water absorbed. The material for the excrement disposal absorbed urine to become gel. The gel exhibited such a viscosity that the gel began to flow slowly when the beaker was inclined. The gel retained urine therein even when the content of the beaker was poured out on a stainless steel mesh. The gel from the composition Nos. 2 and 6 was thrown away into a flush toilet without a splash, and there was no gel left in the beaker. The toilet was flushed easily. Table 1 shows the amount of water absorbed in each composition in 5 minutes.

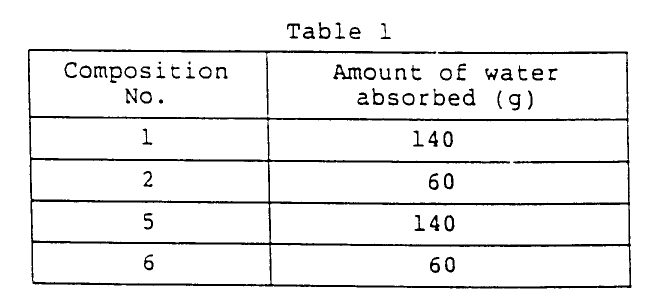

### Test Example 2

Each of the composition Nos. 3 and 7 obtained in Examples was cut into five equal pieces by scissors. Each piece was put into an artificial anus which was made of vinyl plastics, having an inlet pipe 30 mm in diameter and worn by a subject who had loose feces. The material for the excrement disposal gradually absorbed water from feces to form a gel. The gelation proceeded with the elapse of time. The composition in contact with feces lost its shape but did not form undissolved lumps because it is spongy. The feces disposal in an artificial anus was easily carried out owing to the fluidity of the superabsorbent polymer gel and the looseness of feces.

### Test Example 3

Each of the composition Nos. 4 and 8 obtained in Examples was cut into five equal pieces by scissors. Each piece (containing 2g of "Sanwet") was sandwiched between two pieces of cotton nonwoven fabric, 135 × 200 mm, ("Cot-Ace" CXX-1032 and CRS 4015-E14, with one side water-proofed, made by Unitika Ltd.). The assembly was spread on a diaper, and the diaper was put on a one-year old baby. The amount of urine for 3 hours was about 150g, but the baby's hips remained dry to touch. The spongy material for the excrement disposal swelled but the resulting gel did not scatter.

### Test Example 4

Each of the composition Nos. 5, 6 and 8 obtained in Examples was allowed to absorb urine of a healthy adult for gelation. The urine before and after gelation was subjected to the organoleptic test in accordance with the 9 degree ranking indication for comfort or discomfort (Notice No. 76 from the Ministry of Health and Welfare, Environmental Health Bureau, dated December 4, 1990). The results are shown in Table 2.

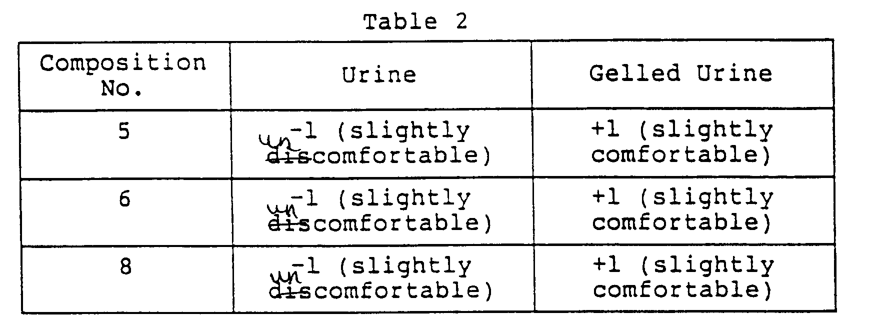

### Test Example 5

The composition No. 7 obtained in Example was mixed with loose feces for gelation. The feces before and after gelation were ranked for discomfort in the same manner as in Test Example 4. The feces before gelation were ranked as -3 (very uncomfortable), whereas the feces after gelation were ranked as -1 (slightly uncomfortable).

### Test Example 6

Each of the compositions Nos. 9 and 10 obtained in Examples was cut into rectangular pieces (40 × 80 mm and 180 × 100 mm) by scissors. For determination of the amount of water absorbed, each composition was immersed in 500g of tap water or 300g of artificial urine for 30 minutes in a container. The resulting slurry was filtered through a 200-mesh wire net and the weight of the filtrate was measured. The amount of water absorbed (in gram) was determined by subtracting the weight of the filtrate from the weight of the water (or urine) which was initially present in the container. The results are shown in Table 3. The compositions Nos. 9 and 10 exhibited a very high water absorption ability.

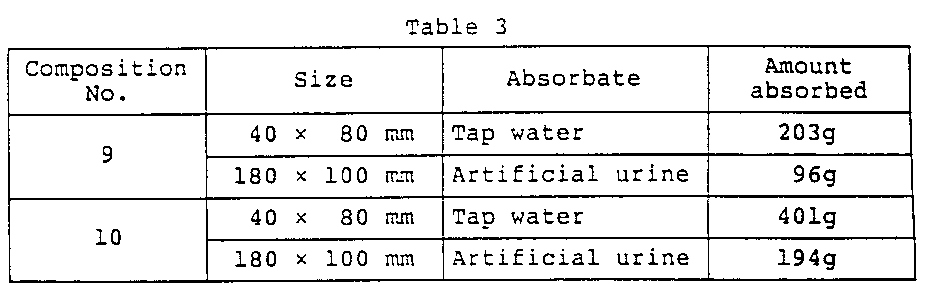

### Test Example 7

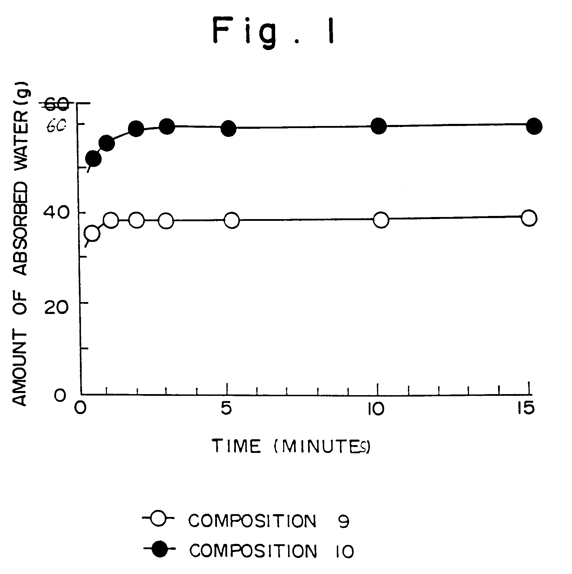

Each of the compositions Nos. 9 and 10 obtained in Examples was cut into pieces (30 × 30 mm) by a cutter knife. The following test was conducted to examine the water absorption ability of each composition with the elapse of time. Each composition was kept in contact with 100g of tap water and the amount of water absorbed was measured after 0.5, 1, 2, 3, 5, 10 and 15 minutes in the same manner as in the Test Example 1. The results are shown in Fig. 1. It is noted that the water absorption was saturated in 1 - 2 minutes. This indicates that the compositions can absorb water very rapidly in the initial stage.

As mentioned above, the composition of the present invention has a spongy structure and can advantageously be used for the excrement disposal because the composition can rapidly absorb water in the initial stage. The composition of the present invention may be supplemented with a filter aid to enhance water absorption ability. The present composition supplemented with a filter aid can easily be molded into a sheet to prepare a spongy superabsorbent sheet. The spongy sheet can rapidly absorb water in the initial stage and keeps excellent water absorption ability, because excellent water absorption ability of the superabsorbent resin is not inhibited. The performance of the superabsorbent sheet differs from that of the superabsorbent polymer alone. That is, the composition does not become undissolved lumps when contacted with loose feces in an artificial anus in case of using the present composition as such or molding the present composition into a superabsorbent sheet for the purpose of the material for the excrement disposal. In addition, the spongy sheet retains its shape until it comes into contact with urine when it is placed in the excrement. Therefore, the sheet remains effective for a long time. Moreover, the sheet can easily be cut into desired size and shape for intended use by a cutter knife or scissors. Since the sheet is water-soluble, the sheet can be thrown away in a flush toilet. The sheet can be used for an artificial anus, artificial bladder, urine glass and night chairs.

The invention is described with reference to the following examples.

### Example 1

The following four components were mixed in a 300-ml beaker: 10g of superabsorbent polymer, "Sumikagel N-100" (crosslinked sodium polyacrylate) from Sumitomo Chemical Co., Ltd.; 1g of crushed pulp (2.5 - 2.8 mm long fiber); 50g of 5% "Paogen EP-15" - containing chloroform, "Paogen EP-15" is a polyalkylene oxide commercially available from Dai-ichi Kogyo Seiyaku Co., Ltd.; and 20g of methyl alcohol.

The resulting slurry mixture was uniformly cast onto a teflon-coated metal vat (160 × 115 mm inside). The vat was vibrated for 10 seconds by a vibrator held in contact with its bottom. The casting was allowed to stand at room temperature for the solvent evaporation. Thus Composition No. 1 was obtained as an approximately 3 mm thick spongy sheet.

### Example 2

The same procedure as in Example 1 was repeated to give a spongy sheet sample (Composition No. 2), except that the superabsorbent polymer was replaced by "Aquareserve AP-150A" (crosslinked sodium polyacrylate), from Nippon Gosei Co., Ltd. and that the fiber was replaced by the cotton yarn (3 - 4 mm long fiber).

### Example 3

The same procedure as in Example 1 was repeated to give a spongy sheet sample (Composition No. 3), except that the amount of methyl alcohol was changed to 10g.

### Example 4

The following four components were mixed in a 300-ml beaker: 10g of a superabsorbent polymer, "Sanwet 1M-1000" (crosslinked starch-acrylate graft copolymer) from Sanyo Chemical Industries, Ltd.; 1g of cotton yarn (3 - 4 mm long fiber); 50g of 5% "Paogen" containing chloroform; and 20g of methyl alcohol.

The resulting slurry mixture was uniformly cast onto a teflon-coated metal vat (160 × 115 mm inside). The vat was vibrated for 10 seconds by a vibrator held in contact with its bottom. The casting was allowed to stand at room temperature for the solvent evaporation. Thus Composition No. 4 was obtained as an approximately 3 mm thick spongy sheet.

### Example 5

Composition No. 5 was prepared by spraying the surface (rich in short fibers) of Composition No. 1 obtained in Example 1, with about 1 ml of eucalyptus oil (from Takasago International Corporation) by a household sprayer, followed by drying by a dryer at 40°C for 1 hour.

### Example 6

The same procedure as in Example 1 was repeated to give Composition No. 6, except that the superabsorbent polymer was replaced by "Aquareserve AP-150A" (crosslinked sodium polyacrylate, product of Nippon Gosei Co., Ltd.), that the fiber was replaced by cotton yarn (3 - 4 mm long fiber), and that a 1:1 mixture of lavender oil and sweet orange oil, commercially avaiable from Takasago International Corporation was used as a deodorant.

### Example 7

The same procedure as in Example 1 was repeated to give a spongy Composition No. 7, except that the amount of methyl alcohol was changed to 10g and that about 1 ml of a 5% dry-distillate of tea plant, "FS-500A" (a product of Shiraimatsu Shinyaku Co., Ltd.) was used as a deodorant.

### Example 8

Composition No. 8 was prepared by spraying the surface (rich in fibers) of Composition No. 4 obtained in Example 4 with about 1 ml of a 1:1 mixture of 3% "Paogen" containing ethyl alcohol and peppermint oil (product of Takasago International Corporation) by a household sprayer, followed by drying at room temperature for 1 hour.

### Example 9

The following four components were mixed in a 1-liter separable flask: 40g of superabsorbent polymer, "Sumikagel N-100" (crosslinked sodium polyacrylate) from Sumitomo Chemical Co., Ltd.; 10g of crushed pulp (2.0 - 2.8 mm long fiber); 150g of diatomaceous earth, "Radiolite #600" (a product of Showa Kagaku Kogyo Co., Ltd.); and 400g of methanol containing 10% "Paogen EP-15" (a product of Dai-ichi Kogyo Seiyaku Co., Ltd.). The resulting slurry mixture was cast onto a silicon-treated, biaxially oriented polyethylene terephthalate (PET) film using a doctor blade ("DP-150" made by Tsugawa Seiki Seisakusho Co., Ltd. ) with the gap being 1.5 mm. The casting was allowed to stand at room temperature for the solvent evaporation. On peeling, Composition No. 9 was obtained as a spongy sheet with a dry touch.

### Example 10

The same procedure as in Example 9 was repeated to give a spongy sheet Composition No. 10, except that the amount of the superabsorbent resin was changed to 100g.

### Industrial Applicability

The present invention provides a composition for a superabsorbent gelling sheet which has a great absorption capacity per unit area, and rapidly absorbs a large amount of water in the initial stage. The sheet can easily be cut into any desired shape. Further, the present invention provides a material for the excrement disposal comprising the composition.

## Claims

1. A composition for superabsorbent gelling sheets which comprises a superabsorbent polymer, fiber, polyalkylene oxide and a filter aid.

2. The composition according to claim 1, wherein the superabsorbent polymer is selected from crosslinked sodium polyacrylate, acrylic acid-vinyl alcohol copolymer, polyvinyl alcohol copolymer, saponified vinyl acetate-methyl acrylate copolymer, saponified vinyl acetate-monomethyl maleate copolymer, saponified isobutylene-maleic acid anhydride copolymer, crosslinked starch-acrylic acid salt graft copolymer, hydrolysed starch-acrylonitrile graft copolymer and crosslinked modified polyethylene oxide.

3. The composition according to claim 1, wherein the fiber is a natural fiber or a synthetic fiber.

4. The composition according to claim 1, wherein the filter aid is selected from the group consisting of diatomaceous earth, activated carbon, talc and pearlite.

5. The composition according to claim 1, wherein the superabsorbent polymer is contained in an amount of 5 - 75 wt%.

6. The composition according to claim 1, wherein the fiber is contained in an amount less than 20 wt%.

7. The composition according to claim 1, wherein the polyalkylene oxide is contained in an amount less than 35 wt%.

8. The composition according to claim 1, wherein the filter aid is contained in an amount more than 0.1 wt%.

## Patentansprüche

1. Zusammensetzung für ein sehr stark absorbierendes gelierendes Blatt, die ein sehr stark absorbierendes Polymer, Faser, Polyalkylenoxid und eine Filterhilfe umfaßt.

2. Zusammensetzung nach Anspruch 1, wobei das sehr stark absorbierende Polymer aus vernetztem Natriumpolyacrylat, Acrylsäure-Vinylalkohol-Copolymer, Polyvinylalkohol-Copolymer, verseiftem Vinylacetat-Methylacrylat-Copolymer, verseiftem Vinylacetat-Monomethylmaleat-Copolymer, verseiftem Isobutylen-Maleinsäureanhydrid-Copolymer, vernetztem Stärke-Acrylsäuresalz-Pfropfcopolymer, hydrolysiertem Stärke-Acrylnitril-Pfropfcopolymer und vernetztem modifiziertem Polyethylenoxid ausgewählt ist.

3. Zusammensetzung nach Anspruch 1, wobei die Faser eine natürliche Faser oder eine synthetische Faser ist.

4. Zusammensetzung nach Anspruch 1, wobei die Filterhilfe aus Kieselerde, Aktivkohle, Talk und Perlit ausgewählt ist.

5. Zusammensetzung nach Anspruch 1, wobei das sehr stark absorbierende Polymer in einer Menge von 5 - 75 Gewichts-% enthalten ist.

6. Zusammensetzung nach Anspruch 1, wobei die Faser in einer Menge von weniger als 20 Gewichts-% enthalten ist.

7. Zusammensetzung nach Anspruch 1, wobei das Polyalkylenoxid in einer Menge von weniger als 35 Gewichts-% enthalten ist.

8. Zusammensetzung nach Anspruch 1, wobei die Filterhilfe in einer Menge von mehr als 0,1 Gewichts-% enthalten ist.

## Revendications

1. Composition pour feuilles gélifiantes superabsorbantes, qui comporte un polymère superabsorbant, des fibres, un poly(oxyalkylène) et un adjuvant de filtration.

2. Composition conforme à la revendication 1, dans laquelle le polymère superabsorbant est choisi parmi un poly(acrylate de sodium) réticulé, un copolymère d'acide acrylique et d'alcool vinylique, un copolymère de type poly(alcool vinylique), un copolymère saponifié d'acétate de vinyle et d'acrylate de méthyle, un copolymère saponifié d'acétate de vinyle et de maléate de monométhyle, un copolymère saponifié d'isobutylène et d'anhydride de l'acide maléique, un copolymère de greffage de sel d'acide acrylique et d'amidon réticulé, un copolymère de greffage d'acrylonitrile et d'amidon hydrolysé, et un poly(oxyéthylène) modifié réticulé.

3. Composition conforme à la revendication 1, dans laquelle les fibres sont des fibres naturelles ou des fibres synthétiques.

4. Composition conforme à la revendication 1, dans laquelle l'adjuvant de filtration est choisi parmi de la terre de diatomées, du charbon actif, du talc et de la perlite.

5. Composition conforme à la revendication 1, qui contient de 5 à 75 % en poids de polymère superabsorbant.

6. Composition conforme à la revendication 1, qui contient moins de 20 % en poids de fibres.

7. Composition conforme à la revendication 1, qui contient moins de 35 % en poids de poly(oxyalkylène).

8. Composition conforme à la revendication 1, qui contient plus de 0,1 % en poids d'adjuvant de filtration.
